# EUROPEAN PATENT APPLICATION

(11) **EP 4 417 977 A1**
(43) Date of publication of application: **21.08.2024**
(21) Application number: 22901223.2
(22) Date of filing: 28.11.2022
(51) Int. Cl.: G01N 35/00

(54) **OUTPUT DEVICE, BLOOD ANALYSIS DEVICE, BLOOD ANALYSIS METHOD, AND PROGRAM**

(30) Priority: 03.12.2021 JP 2021196632
(71) Applicant: HORIBA, Ltd., Minami-ku Kyoto-shi Kyoto 601-8510 (JP)
(72) Inventor: NISHIMORI Masashi, Kyoto-shi, Kyoto 601-8510 (JP); OHASHI Akika, Kyoto-shi, Kyoto 601-8510 (JP); NAKAGAWA Yohei, Kyoto-shi, Kyoto 601-8510 (JP)
(74) Representative: Isarpatent
(86) International application number: PCT/JP2022/043722
(87) International publication number: WO 2023/100790

(57) **Abstract**

An output device includes: a measurement information reception portion configured to receive measurement information including a measurement result on a blood sample; a code information generation portion configured to generate code information by adding the measurement information to browsing destination information indicating a browsing destination at which to browse the measurement information; and an output portion configured to output the code information.

## Description

### Technical Field

The present invention relates to an output device such as a printing device, to a blood analysis apparatus provided with such an output device, to a blood analysis method, and to a program.

### Background Art

A specimen analysis system is known with which measurement data on particles contained in a measurement specimen prepared by adding a reagent to a sample can be output in a form corresponding to output mode information (see, for example, Patent Document 1 identified below). On the other hand, a system is also known with which a user can read with a mobile telephone a two-dimensional code printed on an advertisement flier or the like and access a server based on the read information so that via the server the user can browse information on the product encoded in the two-dimensional code on a Web page (see, for example, Patent Document 2 identified below).

### Citation List

### Patent Literature

Patent Document 1: JP-A-2020-51838
Patent Document 2: Japanese Patent No. 4803815

### Summary of Invention

### Technical Problem

It is preferable that a blood analysis apparatus that automatically performs blood analysis such as blood cell counting can output measurement information on a blood sample (e.g., the result of blood cell counting) in a form printed on a sheet of paper by a printing device such as a printer. This permits the user (e.g., a patient or a medical professional) to see the measurement information on the spot by viewing the sheet that is output.

As an output device incorporated in a blood analysis apparatus, it is preferable to use a compact printing device such as a receipt printer to avoid an increase in the size of the blood analysis apparatus. However, a compact printing device outputs a small sheet of paper, and thus the user finds difficulty reading the measurement information printed on it.

One possible solution to that is to provide a system with which measurement information can be stored on, for example, an external server so that a user can with a personal terminal (such as a smartphone or a tablet) access the server to see on the screen of the personal terminal the measurement information stored on the server. Inconveniently, with this system, for the server to store measurement information, it needs to manage the measurement information, and this increases the burden on the server.

Moreover, the sheet of paper that a compact printing device outputs is small and only has a small space for printing. It is therefore difficult to print on it additional information (e.g., advice on a diet) other than measurement information to present it to the user.

Devised to solve the problems mentioned above, the present invention is aimed at providing an output device that can present a user with measurement information on a blood sample with no increase in the size of the output device and no increase in the burden on a server and that permits the user to see additional information, to provide a blood analysis apparatus incorporating such an output device, to provide a blood analysis method, and to provide a program.

### Solution to Problem

According to one aspect of the present invention, an output device includes: a measurement information reception portion configured to receive measurement information including a measurement result on a blood sample; a code information generation portion configured to generate code information by adding the measurement information to browsing destination information indicating a browsing destination at which to browse the measurement information; and an output portion configured to output the code information.

According to another aspect of the present invention, a blood analysis apparatus includes: the output device described above; and a blood analysis portion configured to acquire the measurement information to be fed to the measurement information reception portion in the output device.

According to yet another aspect of the present invention, a blood analysis method involves: a measurement information acquisition step of acquiring measurement information including a measurement result on a blood sample; a measurement information reception step of receiving the measurement information; a code information generation step of generating code information by adding the measurement information to browsing destination information indicating a browsing destination at which to browse the measurement information; and an output step of outputting the code information.

According to a further aspect of the present invention, a program makes a computer execute: a measurement information reception step of receiving measurement information including a measurement result on a blood sample; a code information generation step of generating code information by adding the measurement information to browsing destination information indicating a browsing destination at which to browse the measurement information; and an output step of outputting the code information.

### Advantageous Effects of Invention

According to the present invention, it is possible to present a user with measurement information on a blood sample with no increase in the size of an output device and no increase in the burden on a server, and to permit the user to see additional information.

### Brief Description of Drawings

[Fig. 1] is a perspective view showing an exterior construction of a blood analysis apparatus according to one embodiment of the present invention.
[Fig. 2] is a block diagram showing a detailed configuration of the blood analysis apparatus.
[Fig. 3] is an illustrative diagram schematically showing a procedure of accessing an external site from a user's personal terminal based on information output from an output device in the blood analysis apparatus.
[Fig. 4] is a flow chart showing a sequence of operation on the blood analysis apparatus.
[Fig. 5] is an illustrative diagram showing a detailed configuration of a control device in the blood analysis apparatus.
[Fig. 6] is a flow chart showing a sequence of operation in the control by the control device for switching display screens on an information display portion.
[Fig. 7] is a flow chart showing the above sequence of operation.
[Fig. 8] is an illustrative diagram schematically showing an example of display screens on the information display portion.
[Fig. 9] is a perspective view showing another construction of the blood analysis apparatus.

### Description of Embodiments

An illustrative embodiment of the present invention will be described below with reference to the accompanying drawings. In the following description, a "user" is mainly assumed to be a medical professional who handles a blood analysis apparatus incorporating an output device according to the present invention but may be a patient who is presented with the results of blood analysis by a medical professional.

### [ 1. Outline of a Blood Analysis Apparatus ]

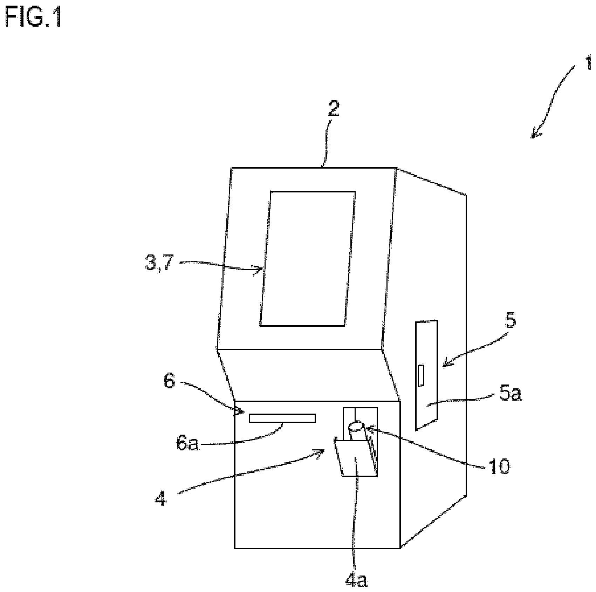

First, a blood analysis apparatus to which an output device according to the embodiment is applicable will be described. Fig. 1 is a perspective view showing the exterior construction of the blood analysis apparatus 1. The blood analysis apparatus 1 includes an information display portion 3 in an upper part of the front face of an apparatus body 2. The information display portion 3 is configured with a liquid crystal display device and displays the results of blood analysis and the like.

In a lower part of the apparatus body 2, a sample container loading portion 4 is provided. Opening a cover 4a of the sample container loading portion 4, then setting a sample container 10 holding a blood sample in it, and then closing the cover 4a permits the sample container 10 to be loaded in the apparatus body 2. The sample container 10 is loaded in the apparatus body 2 with an adaptor that suits the type of the sample container 10 attached to it.

In a side part of the apparatus body 2, a reagent container loading portion 5 is provided. Opening a door 5a of the reagent container loading portion 5 permits the refilling with a reagent (e.g., a reagent for immunoassay) used in blood analysis.

The blood analysis apparatus 1 incorporates an output device 6. The output device 6 is a compact printer (printing device) and discharges through a discharge port 6a a sheet of paper having blood analysis results (measurement results) printed on it. While in Fig. 1 the discharge port 6a is provided in the front face of the apparatus body 2, it may be provided in the top face of the apparatus body 2 or elsewhere.

The output device 6 may be configured to be provided outside the blood analysis apparatus 1 and connected to the blood analysis apparatus 1 so as to be able to communicate with it on a wired or wireless basis. The output device 6 will be described in detail later.

The information display portion 3 mentioned above includes, overlaid on it, an input portion 7 such as a touch panel. The input portion 7 accepts the input of various instructions from a user (e.g., a medical professional). The input portion 7 may be configured with mechanical push buttons that are provided elsewhere than in the information display portion 3.

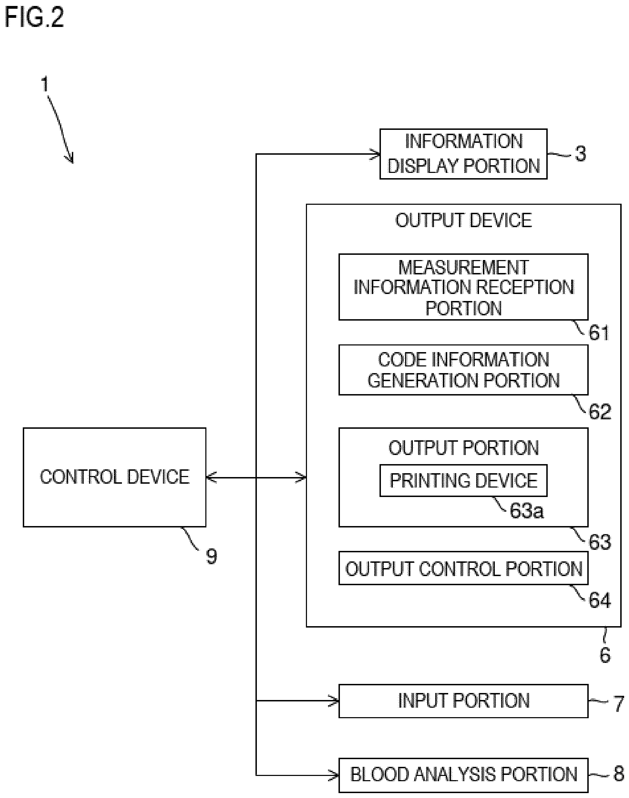

Fig. 2 is a block diagram showing a detailed configuration of the blood analysis apparatus 1. The blood analysis apparatus 1 includes, in addition to the information display portion 3, the output device 6, and the input portion 7 mentioned above, a blood analysis portion 8 and a control device 9.

The blood analysis portion 8 performs blood analysis as by counting the number of blood cells contained in a blood sample. The blood analysis portion 8 includes a plurality of chambers (not illustrated) that receive a blood sample sucked through a nozzle (not illustrated) from the sample container 10 (see Fig. 1) and discharged into them. Inside a chamber for blood cell counting, a counting device is provided. The counting device can carry out, according to the type of blood cells to be counted, various measurement methods including those based on impedance, flow cytometry, and focused flow impedance. Examples of the types of blood cells to be counted include not only red blood cells but also white blood cells (basophils, lymphocytes, monocytes, neutrophils, eosinophils).

The chambers mentioned above may further include those that suit other measurement items such as CRP (C-reactive protein) value, hemoglobin amount, and hemoglobin concentration.

The measurement results of blood analysis in the blood analysis portion 8 are fed as measurement information from the blood analysis portion 8 to the output device 6.

The control device 9 is configured with, for example, a central arithmetic processor (computer) generally called a CPU (central processing unit) and controls the operation of different parts in the blood analysis apparatus 1.

### [ 2. Details of Output Device ]

Next, the output device 6 will be described in detail. The output device 6 includes a measurement information reception portion 61, a code information generation portion 62, an output portion 63, and an output control portion 64. The output control portion 64 is configured with a CPU that controls different parts in the output device 6. The control device 9 mentioned above may be shared to function as the output control portion 64.

The measurement information reception portion 61 receives measurement information fed from the blood analysis portion 8, that is, measurement results on a blood sample. For example, measurement information such as a white blood cell count, a red blood cell count, and a CRP value are fed from the blood analysis portion 8 to the measurement information reception portion 61 and are received by the measurement information reception portion 61. This measurement information reception portion 61 is configured with a connector to which leads leading to the blood analysis portion 8 are connected.

The blood analysis portion 8 and the measurement information reception portion 61 may be configured to communicate with each other on a wireless basis within the blood analysis apparatus 1. In that case, the measurement information reception portion 61 is configured with a communication interface that exchanges information.

The code information generation portion 62 generates code information by adding the measurement information received by the measurement information reception portion 61 to access destination information (URL, uniform resource locator) for access to a Web site on a server. The code information is configured as, for example, a two-dimensional code such as QR code (registered trademark), and may be configured as a one-dimensional code such as a bar code. The code information generation portion 62 may generate the code information by encrypting the measurement information and then adding it to the access destination information. Access destination information and encrypted measurement information can be understood to be access destination information in its broader sense.

The output portion 63 outputs the code information generated by the code information generation portion 62. This output portion 63 is configured to include a printing device 63a. That is, the printing device 63a prints the code information on a sheet of paper. As mentioned above, the sheet is discharged through the discharge port 6a (see Fig. 1).

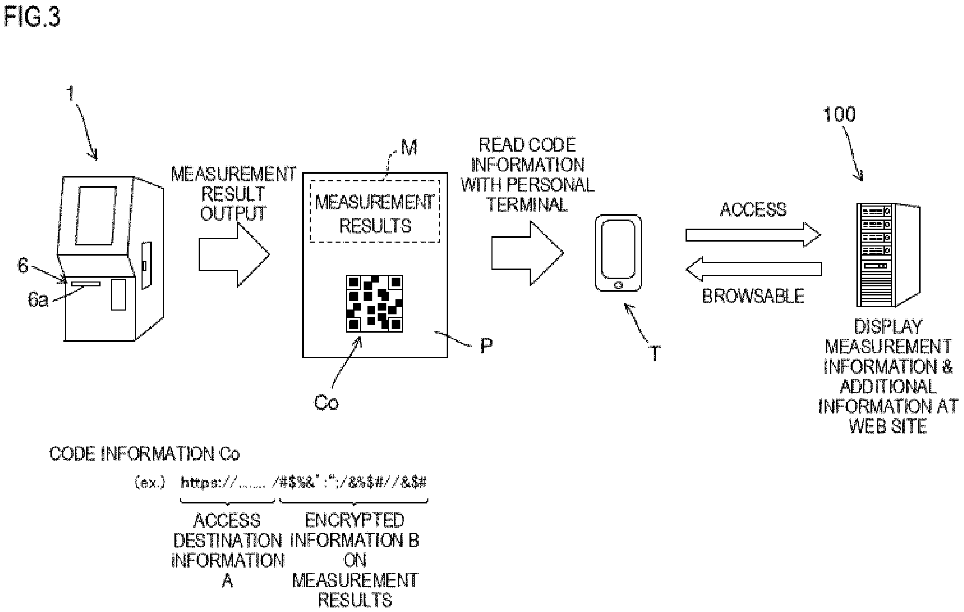

Fig. 3 schematically shows a procedure of accessing a server 100 from a personal terminal T of a user (e.g., patient) based on information printed on a sheet of paper P output from the output device 6 (printing device 63a). Usable as the personal terminal T is, for example, a smartphone, a tablet terminal, a personal computer, or the like.

First, the printing device 63a in the output portion 63 prints, on a sheet P of paper, code information Co generated in the code information generation portion 62 along with measurement information M. Here, the measurement information M is information conveying measurement results (e.g., a blood cell count and the like) acquired in the blood analysis portion 8 (see Fig. 2); the code information Co is information resulting from encoding (e.g., in a form of a two-dimensional code) access destination information A (URL) for a Web site on the server 100 along with encrypted information B resulting from encrypting the measurement information M mentioned above. Here, the measurement information M may include information on flags, which will be described later.

Next, a user (e.g., patient), using a personal terminal T that he or she owns, reads the code information Co printed on the sheet P. In this way, based on the access destination information A included in the code information Co, the user can access the server 100 (Web site) from the personal terminal T.

When the personal terminal T accesses the server 100, the server 100 decodes the encrypted information B included in the code information Co and thereby the measurement results are retrieved. Thus the server 100 can generate additional information based on the acquired measurement results and display, at a Web site on the server 100, the additional information along with the measurement results. That is, by accessing the server 100 from the personal terminal T and communicating between the personal terminal T and the server 100, the user can see, on the display screen of the personal terminal T, what is presented at the Web site (measurement results and additional information). Here, examples of the additional information include advice on a diet, presence or absence of a sign of a particular disease, things to be noted in daily life, and advice for prevention of diseases, that is, information that the user cannot grasp directly from the measurement information alone.

As described above, a user can, by accessing the server 100 from the personal terminal T, see measurement information, that is, the measurement results on the user's blood sample, along with additional information. Thus the output device 6 does not need to be equipped with a function of generating the additional information. Nor does the output portion 63 need to be configured as a large-size (high-spec) one that can output the additional information. It is thus possible to provide the user with measurement information while keeping the output portion 63 compact and avoiding an increase in the size of the output device 6.

Accessing the server 100 from the personal terminal T results in the measurement information included in the code information Co being sent from the personal terminal T to the server 100. Thus the server 100 does not need to hold (store) the measurement information in advance. This helps reduce the management burden on the server 100.

Even if the output device 6 is so compact that it cannot output additional information in addition to measurement information M, configuring the output device 6 to output code information Co permits the user to read the code information Co with the personal terminal T and access the server 100 to see additional information on the display screen of the personal terminal T.

The code information generation portion 62 in the output device 6 encrypts the measurement information and then adds it to the access destination information A. With this configuration, if for some cause the measurement information is leaked in the process of the personal terminal T accessing the server 100, it is difficult for a third party to crack the measurement information, and this helps protect measurement information (individual information).

The output portion 63 outputs (on the sheet p), along with the code information Co, the measurement information M received by the measurement information reception portion 61. Thus the user can, based on the measurement information M output from the output portion 63, learn the blood condition on the spot (immediately).

The output portion 63 includes the printing device 63a that prints the code information Co on a sheet of paper P. With the code information Co printed by the printing device 63a, even at a place remote from the output device 6, the user can read the code information Co printed on the sheet P with the personal terminal T and access the server 100 based on the access destination information A included in the code information Co to see measurement information and like on the display screen of the personal terminal T. This is particularly effective if the user is a patient who is not usually present around the output device 6.

The blood analysis apparatus 1 according to the embodiment includes the output device 6 and the blood analysis portion 8 described above. The blood analysis portion 8 analyzes a blood sample to acquire measurement information to be fed to the measurement information reception portion 61 in the output device 6. The measurement information thus acquired by the blood analysis portion 8 is received by the output device 6 to output code information Co. So configured, the blood analysis apparatus 1 provides the effects described above.

The user's personal terminal T may have an application installed on it that permits the user to browse measurement information. In that case, in the personal terminal T, information (movement destination information) for moving to a folder that is the destination of the browsing of measurement information by the application mentioned above may be used instead of the access destination information A described above. That is, the movement destination information may be fed to the printing device 63a in the output portion 63 in advance. With this configuration, when the user reads the code information Co printed on a sheet P with the personal terminal T, the personal terminal T accesses (moves to) the above-mentioned folder based on the movement destination information included in the code information Co so that, in that folder, the above-mentioned application decodes the encrypted information B included in the code information Co. This permits the user to browse the measurement information in that folder with the personal terminal T.

Thus, if the access destination information and the movement destination information mentioned above are referred to collectively as browsing destination information, the embodiment may be understood to be configured as follows. The code information generation portion 62 may generate code information Co by adding measurement information to browsing destination information. The code information generation portion 62 may encrypt the measurement information and then add it to the browsing destination information. Moreover, the browsing destination information mentioned above may include access destination information A for access to an external site (Web site). Or the browsing destination information mentioned above may include movement destination information for moving to a folder that is the destination of the browsing of measurement information by an application on a personal terminal.

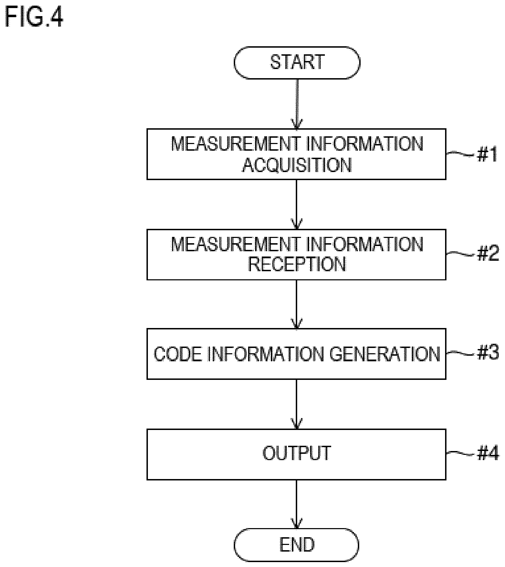

Fig. 4 is a flow chart showing the sequence of operation on the blood analysis apparatus 1 described above. The blood analysis apparatus 1 can be understood to perform, as shown in Fig. 4, a measurement information acquisition step (#1), a measurement information reception step (#2), a code information generation step (#3), and an output step (#4). That is, the blood analysis method carried out by the blood analysis apparatus 1 includes a measurement information acquisition step (#1), a measurement information reception step (#2), a code information generation step (#3), and an output step (#4).

In #1, the blood analysis portion 8 acquires measurement information including measurement results on a blood sample. In #2, the measurement information acquired by the blood analysis portion 8 in #1 and fed from the blood analysis portion 8 is received by the measurement information reception portion 61. In #3, the measurement information received by the measurement information reception portion 61 is added to browsing destination information to generate code information Co. In #4, the code information generated in #3 is output (e.g., printed on a sheet of paper P).

Thus a user can, with a personal terminal T, read the code information Co (printed on the sheet P) and access, based on the browsing destination information (e.g., access destination information A) included in the code information Co, a server 100 from the personal terminal T. Accordingly, with the blood analysis method described above, it is possible to present the user with measurement information while avoiding an increase in the size of a output device 6 and otherwise obtain effects similar to those offered by the embodiment described above.

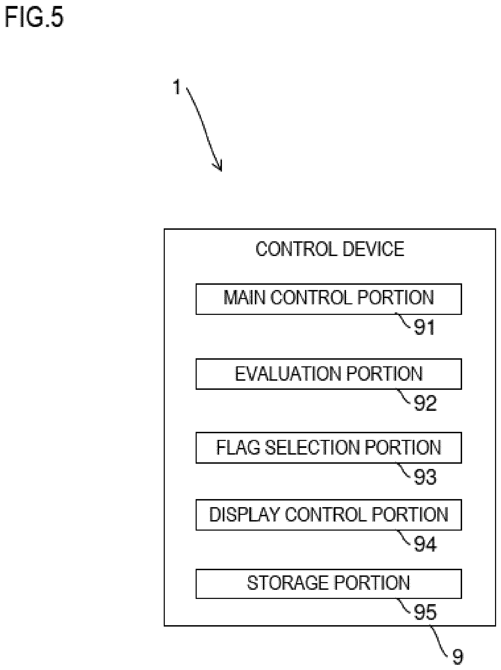

The blood analysis apparatus 1 according to the embodiment may be configured to include a computer having an operation program (application software) installed on it. The computer (e.g., the control device 9 as a CPU) can read and execute the just-mentioned program to make different parts in the blood analysis apparatus 1 operate to perform the operation (steps) described above. Such a program can be obtained, for example, by being downloaded from outside via a network and stored in a storage portion 95 (see Fig. 5) within the control device 9. Or the program can be recorded on a computer-readable recording medium such as a CD-ROM (compact disk-read only memory) to be read from the recording medium by a reading portion (not illustrated) to be stored in the storage portion 95. That is, the program according to the embodiment is a program for making a computer execute a measurement information reception step of receiving measurement information including measurement results on a blood sample, a code information generation step of generating code information by adding the measurement information to browsing destination information, and an output step of outputting the code information. The recording medium mentioned above is a computer-readable recording medium having the program mentioned above recorded on it.

### [ 3. Controlling Display on the Information Display Portion ]

Fig. 5 is an illustrative diagram showing a detailed configuration of the control device 9 in the blood analysis apparatus 1 according to the embodiment. The display of various kinds of information on the information display portion 3 (see Figs. 1 and 2) of the blood analysis apparatus 1 is controlled by the control device 9. The control device 9 includes a main control portion 91, an evaluation portion 92, a flag selection portion 93, a display control portion 94, and a storage portion 95.

The main control portion 91 controls the operation of different parts in the blood analysis apparatus 1. The evaluation portion 92 judges whether the measurement results included in the measurement information acquired by the blood analysis portion 8 (see Fig. 2) fall within normal ranges.

If a measurement result falls outside a normal range, the flag selection portion 93 selects, out of a plurality of flags, the flag corresponding to that measurement result. For example, the flag selection portion 93 selects, if a measurement result on white blood cells is lower than the normal range, a flag "L" and, if it is higher than the normal range, a flag "H". Here, the flag selected by the flag selection portion 93 is selected out of a plurality of previously prepared flags according to the item measured in the blood analysis portion 8.

The display control portion 94 controls display on the information display portion 3. In particular, the display control portion 94 switches display screens on the information display portion 3 according to instructions that the user inputs on the input portion 7 (see Figs. 1 and 2).

The storage portion 95 is a memory that stores an operation program for the control device 9 as well as various kinds of information, and is configured to include a ROM (read-only memory), a RAM (a random-access memory), a non-volatile memory, and the like. The storage portion 95 may be provided outside the control device 9.

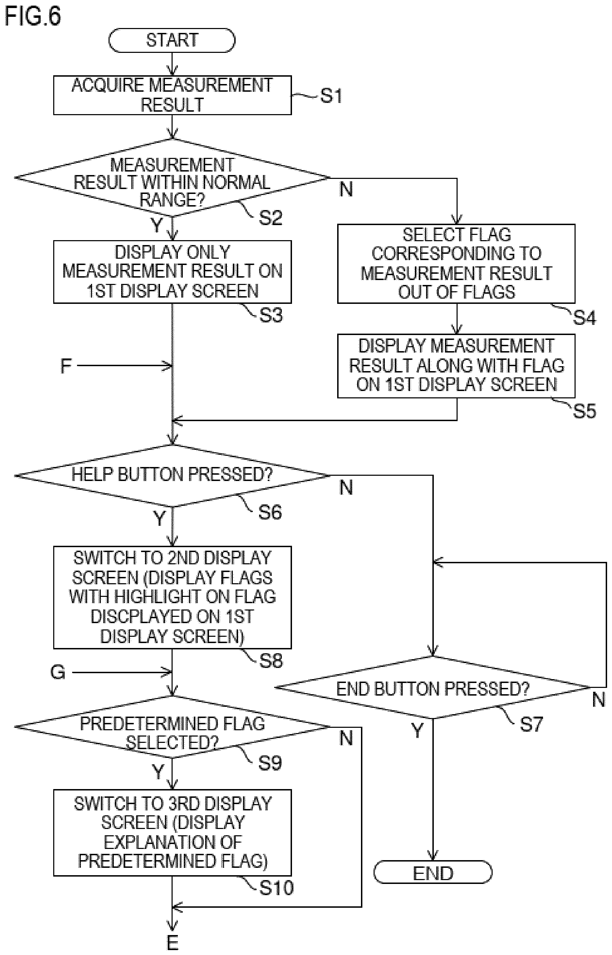

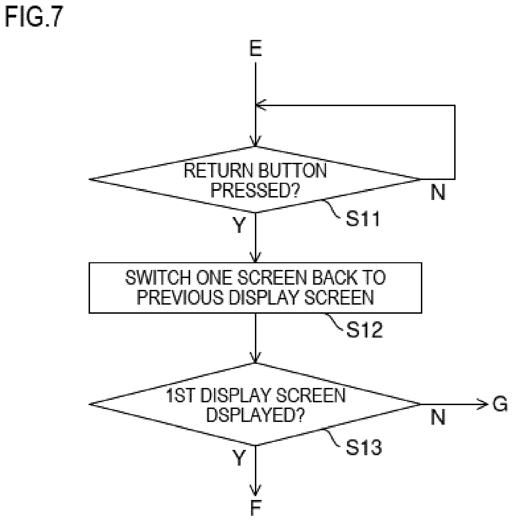

Figs. 6 and 7 are flow charts showing the sequence of operation performed by the control device 9 to switch display screens on the information display portion 3. How the operation proceeds will now be described in detail. In the following description, a part of the input portion 7 configured with a touch panel that corresponds to a predetermined display region on the information display portion 3 will referred to also as a "button". For example, a part of the input portion 7 that corresponds to a region where "help" is displayed on the information display portion 3 is referred to also as the "help button".

When the blood analysis portion 8 acquires measurement results on a blood sample (S1), the evaluation portion 92 in the control device 9 checks, for each measurement item in the blood analysis portion 8, whether the measurement result falls within a normal range (S2). If in S2 the measurement result falls within the normal range, the display control portion 94 makes the information display portion 3 display only the measurement result (S3).

By contrast, if, in S2 the measurement result falls outside the normal range, the flag selection portion 93 selects, out of a plurality of flags, the flag that corresponds to the above-mentioned measurement result (S4). The display control portion 94 then makes the information display portion 3 display the measurement result along with the flag selected in S4 (S5).

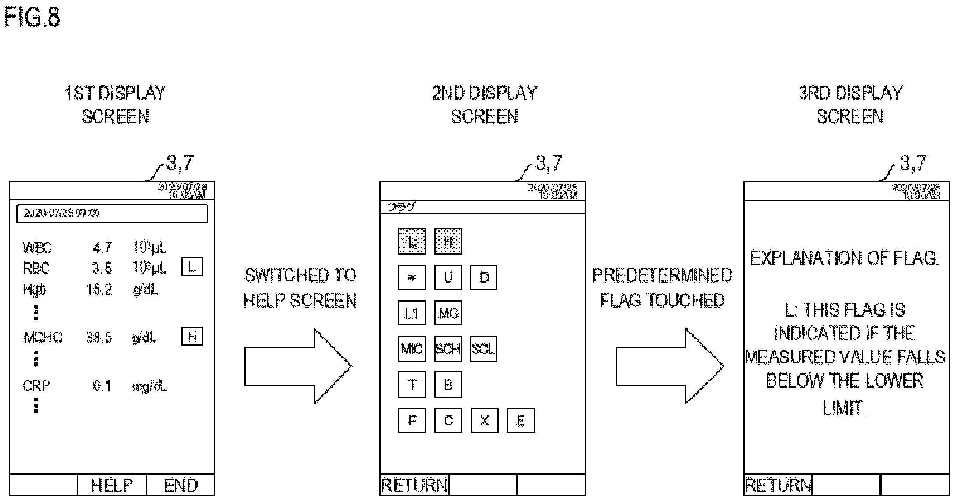

Fig. 8 schematically shows an example of display screens on the information display portion 3. The display screen at left in the diagram (in the following description, referred to also as the first display screen), for WBC (white blood cell count), Hgb (hemoglobin amount), and CRP (C-reactive protein), only measurement results are displayed, and this indicates that those values fall within the normal ranges. By contrast, for RBC (red blood cell count), the measurement result is displayed along with a flag "L", and this indicates that the value is lower than the normal range. On the other hand, for MCHC (hemoglobin concentration), the measurement result is displayed along with a flag "H", and this indicates that the value is higher than the normal range.

The first display screen also has display regions labeled "help" and "end". If the user wants to be presented with a detailed explanation of each of the plurality of flags, the user can press the help button, and this results in the display screen on the information display portion 3 being switched as will be described later. On the other hand, when finishing the display on the information display portion 3, the user can press the end button.

After S3 or S5, the display control portion 94 checks whether the help button is pressed on the first display screen (S6). If in S6 the help button is not pressed, the display control portion 94 then checks whether the end button is pressed on the same first display screen (S7). If in S7 the end button is pressed, the entire sequence of operation is ended.

By contrast, if in S6 the help button is pressed, the display control portion 94 switches the display screen on the information display portion 3 from the first display screen to the second display screen (S8). On the second display screen, the display control portion 94 makes the information display portion 3 selectably display a plurality of flags and, for a flag that has been displayed on the first display screen, makes the information display portion 3 display it with an appearance different from the other flags.

For example, on the second display screen shown at middle in Fig. 8, the flags "L" and "H", which have been displayed on the first display screen, are displayed emphasized as compared with the other flags. In Fig. 8, hatched flags are those displayed emphasized (to be conspicuous) and unhatched flags are those displayed unemphasized (to be inconspicuous). On the second display screen, any of the flags, regardless of whether they are displayed to be conspicuous or inconspicuous, can be pressed to be selected.

Next, the display control portion 94 checks on the second display screen whether the user has selected a predetermined flag, that is, whether the button of a predetermined flag has been pressed (S9). If in S9 the button of a predetermined flag has been pressed, the display control portion 94 switches the display screen on the information display portion 3 from the second display screen to the third display screen (S10).

On the third display screen, the display control portion 94 makes the information display portion 3 display a detailed explanation of the flag selected on the second display screen. The third display screen at right in Fig. 8 is an example seen when the flag "L" is selected on the second display screen and a detailed explanation of the flag "L" is being displayed.

It is here assumed that the data of the explanation of each flag is previously stored in the storage portion 95. The display control portion 94 can, by extracting the data of the flag selected in S9 from the storage portion 95, display a detailed explanation of the selected flag on the information display portion 3.

Next, the display control portion 94 checks on the third display screen whether a return button is pressed (S11). If in S11 the return button is pressed, the display control portion 94 switches the display screen on the information display portion 3 one screen back to the second display screen (S12). Since the second display screen is not the first display screen (in S13, No), after that, an advance is made to S9, where operation similar to that described above is performed.

By contrast, if in S9 no button for a predetermined flag is pressed, also then an advance is made to S11, where the display control portion 94 checks whether the return button is pressed on the second display screen. If in S11 the return button is pressed, the display control portion 94 switches the display screen on the information display portion 3 one screen back to the first display screen (S12). Then an advance is made via S13 to S6, where operation similar to that described above is performed.

As described above, the information display portion 3, if a measurement result on the blood sample in the blood analysis portion 8 falls within a normal range, displays the measurement result and, if the measurement result falls outside the normal range, displays the measurement result along with a flag (S3, S5). When a display screen on the information display portion 3 that displays at least a measurement result is taken as a first display screen, if the help button is pressed on the input portion 7, that is, if an instruction to request display of an explanation of a plurality of flags is entered, the display control portion 94 switches from the first display screen to a second display screen that allows selection of one of the plurality of flags (S6, S8).

In this embodiment, on the second display screen, the display control portion 94 displays, out of the plurality of flags, a flag displayed on the first display screen with an appearance different from the other flags. Thus, on the second display screen, the flag displayed on the first display screen can be displayed highlighted. Thus, on the second display screen, the user can easily grasp which is the flag displayed on the first display screen, that is, the flag added to the measurement result.

In particular, on the second display screen, the display control portion 94 has, out of the plurality of flags, the flag displayed on the first display screen displayed emphasized as compared with the other flags (see Fig. 8). Thus, on the second display screen, the flag displayed on the first display screen is reliably displayed highlighted. This makes it easier for the user to recognize that flag.

On the second display screen, instead of a flag displayed on the first display screen being displayed emphasized as compared with the other flags, the flag displayed on the first display screen can be displayed in thicker letters, in larger letters, in decorated letters, in a different color, in a different font, or otherwise so that the flag displayed on the first display screen can be displayed highlighted.

After switching from the first display screen to the second display screen, on recognizing a predetermined flag to be selected on the input portion 7, the display control portion 94 switches from the second display screen to a third display screen that displays an explanation of the predetermined flag selected (S9, S10). Thus, the user can, on the second display screen, select the above-mentioned flag on the input portion 7 and, on the third display screen, have the explanation of that flag displayed to immediately see the explanation.

The output portion 63 in the output device 6 can be configured to include, instead of or in addition to the printing device 63a, a display device that displays code information Co. Also in that case, the user can access an external site by reading the code information Co displayed on that display device with the personal terminal T. The information display portion 3 described above may be shared to function as a display device that displays the code information Co. The printing device 63a may be a printing device that is externally connected to the output device 6. In that case, as the externally connected printing device it is possible to use a user's own printing device.

### [ 4. Another Configuration of a Blood Analysis Apparatus ]

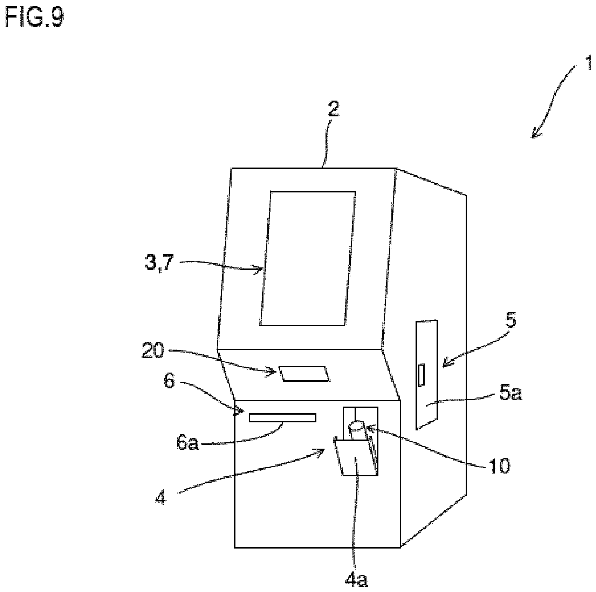

Fig. 9 is a perspective view showing another configuration of the blood analysis apparatus 1 according to the embodiment. In the front face of the apparatus body 2, between the information display portion 3 and the sample container loading portion 4, an information reding portion 20 may be provided. The information reding portion 20 is configured with, for example, a bar code reader, and reads peripheral information attached to the sample container 10.

Here, the peripheral information includes registration information such as a registration number of the patient who presented blood, information on a reagent in the sample container 10 (e.g., whether an anticoagulant is contained), and identification information unique to each sample container 10 for distinguishing individual sample containers 10. The identification information includes manufacturing information such as the manufacturing number and the manufacturer of the sample container 10 and information on the type of the sample container 10 (standard blood collection tube, blood microcollection tube, special container, etc.). While at least part of the peripheral information mentioned above is represented by a bar code (one-dimensional code), it may be represented by, instead of a bar code, a two-dimensional code such as a QR code (a registered trademark).

Incorporating the information reding portion 20 in the blood analysis apparatus 1, as compared with having to use an externally connected reading portion (e.g., a bar code reader), permits the information reding portion 20 to read information even from a small space, or with the sample container 10 held in one hand.

While embodiments of the present invention have been described above, they are in no way meant to limit the scope of the present invention, which can be carried out with any extensions or modifications made without departure from the spirit of the invention.

### Industrial Applicability

The present invention finds applications in, for example, output devices in blood analysis apparatuses, and in blood analysis apparatuses.

### Reference Signs List

- 1: blood analysis apparatus
- 3: information display portion
- 6: output device
- 7: input portion
- 8: blood analysis portion
- 10: sample container
- 20: information reding portion
- 61: measurement information reception portion
- 62: code information generation portion
- 63: output portion
- 63a: printing device
- 92: evaluation portion
- 93: flag selection portion
- 94: display control portion
- A: access destination information (browsing destination information)
- Co: code information
- M: measurement information

## Claims

1. An output device comprising:
a measurement information reception portion configured to receive measurement information including a measurement result on a blood sample;
a code information generation portion configured to generate code information by adding the measurement information to browsing destination information indicating a browsing destination at which to browse the measurement information; and
an output portion configured to output the code information.

2. The output device according to claim 1, wherein
the code information generation portion is configured to encrypt the measurement information and then add it to the browsing destination information.

3. The output device according to claim 1 or 2, wherein
the browsing destination information includes access destination information for access to an external site.

4. The output device according to any one of claims 1 to 3, wherein
the output portion is configured to output the measurement information along with the code information.

5. The output device according to any one of claims 1 to 4, wherein
the output portion includes a printing device configured to print the code information.

6. A blood analysis apparatus comprising:
the output device according to any one of claims 1 to 5; and
a blood analysis portion configured to acquire the measurement information to be fed to the measurement information reception portion in the output device.

7. The blood analysis apparatus according to claim 6, further comprising:
an evaluation portion configured to check whether the measurement result included in the measurement information acquired by the blood analysis portion falls within a normal range;
a flag selection portion configured to select, out of a plurality of flags, a flag corresponding to the measurement result if the measurement result falls outside the normal range;
an information display portion configured to display, if the measurement result falls within the normal range, the measurement result and, if the measurement result falls outside the normal range, the measurement result along with the flag;
an input portion configured to receive an instruction input by a user; and
a display control portion configured to switch display screens on the information display portion according to the input instruction,
wherein
when a display screen on the information display portion that displays at least the measurement result is taken as a first display screen,
if an instruction requesting display of an explanation of the plurality of flag is input to the input portion, the display control portion switches from the first display screen to a second display screen that allows selection of one of the plurality of flags, and
on the second display screen, the display control portion makes the information display portion display, out of the plurality of flags, the flag displayed on the first display screen with an appearance different from other flags.

8. The blood analysis apparatus according to claim 7, wherein
on the second display screen, the display control portion has, out of the plurality of flags, the flag displayed on the first display screen displayed emphasized as compared with the other flags.

9. The blood analysis apparatus according to claim 7 or 8, wherein
after switching from the first display screen to the second display screen, on recognizing a predetermined flag to be selected on the input portion, the display control portion switches from the second display screen to a third display screen that displays an explanation of the predetermined flag selected.

10. The blood analysis apparatus according to any one of claims 6 to 9, further comprising:
an information reading portion configured to read peripheral information attached to a sample container that holds the blood sample.

11. A blood analysis method comprising:
a measurement information acquisition step of acquiring measurement information including a measurement result on a blood sample;
a measurement information reception step of receiving the measurement information;
a code information generation step of generating code information by adding the measurement information to browsing destination information indicating a browsing destination at which to browse the measurement information; and
an output step of outputting the code information.

12. A program for making a computer execute:
a measurement information reception step of receiving measurement information including a measurement result on a blood sample;
a code information generation step of generating code information by adding the measurement information to browsing destination information indicating a browsing destination at which to browse the measurement information; and
an output step of outputting the code information.
